# EUROPÄISCHE PATENTANMELDUNG

(11) **EP 0 053 096 A1**
(43) Veröffentlichungstag der Anmeldung: **02.06.1982**
(21) Anmeldenummer: 81810455.6
(22) Anmeldetag: 16.11.1981
(51) Int. Cl.: C07D 307/86, C07C 125/067, C07D 231/20, C07D 239/47, C07D 249/12, C07D 317/24, C07D 407/02, A01N 47/18, A01N 47/22

(54) **Unsymmetrische Bis-Carbamate, Verfahren zu ihrer Herstellung und ihre Verwendung in der Schädlingsbekämpfung**

(30) Priorität: 20.11.1980 CH 8590/80; 30.12.1980 CH 9643/80; 25.09.1981 CH 6206/81
(71) Anmelder: CIBA-GEIGY AG, 4002 Basel (CH)
(72) Erfinder: Drabek, Jozef, Dr., CH-4104 Oberwil (CH); Böger, Manfred, CH-7858 Weil am Rhein 5 (CH)

(57) **Zusammenfassung**

Unsymmetrische Bis-Carbamate der Formel 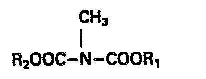 worin R, eine Phenyl-, Dihydrobenzofuranyl-, Naphthyl-, Pyrimidyl-, Pyraaolyl-, Triazolyl-, Chinolinyl- oder Tetrahydrochinolinylgruppe und R₂ eine Benzylgruppe bedeuten.

Verfahren zu ihrer Herstellung und ihre Verwendung in der Schädlingsbekämpfung werden beschrieben.

## Beschreibung

Wegen ihrer Wirkung bevorzugt sind Verbindungen der Formel I, worin R₁ 2,2-Dimethyl-2,3-dihydrobenzofuranyl, Naphthyl, unsubstituiertes oder ein-oder mehrfach durch Cₗ₋C₄₋Alkyl, C₁-C₄-Alkoxy, C₁-C₄-Alkoxy-C₁-C₄-Alkyl, C₁-C₄-Alkylthio-C₁-C₄-Alkyl, Dimethylamino, 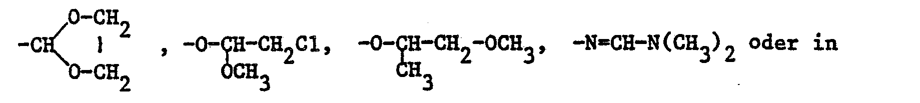 2,3-Stellung durch 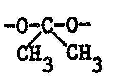 substituiertes Phenyl und R₂ unsubstituiertes oder ein- oder mehrfach durch Halogen, C₁-C₄-Alkyl, C₁-C₄-Alkoxy, Nitro oder in 2,3-Stellung durch -0-CH₂-0- substituiertes Benzyl bedeuten.

Insbesondere bevorzugt sind Verbindungen der Formel I, worin R₁ 2,2-Dimethyl-2,3-dihydrobenzofuranyl, Naphthyl, unsubstituiertes oder ein-, zwei- oder dreifach durch C₁-C₄-Alkyl, C₁-C₄-Alkoxg, Aethylthiomethyl, Aethoxymethyl, Dimethylamino, 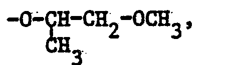 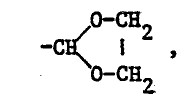 -N=CH-N(CH₃)₂ oder in 2,3-Stellung durch 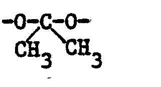 substituiertes Phenyl und R₂ unsubstituiertes oder ein-oder mehrfach durch Halogen, Nitro, Methyl, Methoxy oder in 2,3-Stellung durch -O-CH₂-O-substituiertes Benzyl bedeuten.

Ganz besonders bevorzugt sind aber Verbindungen der Formel I, worin R₁ 2,2-Dimethyl-2,3-dihydrobenzofuranyl und.

R₂ unsubstituiertes oder einfach durch Chlor, Methyl, Methoxy, Nitro oder in 2,3-Stellung durch -O-CH₂-O- substituiertes Benzyl bedeuten.

Die vorliegende Erfindung betrifft unsymmetrische Bis-Carbamate, Verfahren zu ihrer Herstellung und ihre Verwendung in der Schädlingsbekämpfung.

Die unsymmetrischen Bis-Carbamate haben die Formel 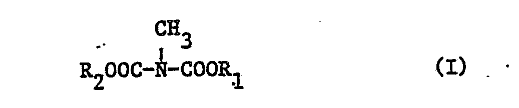 worin R₁ eine Phenyl-, Dihydrobenzofuranyl-, Naphthyl-, Pyrimidyl-, Pyrazolyl-, Triazolyl-, Chinolinyl- oder Tetrahydrochinolinylgruppe und R₂ eine Benzylgruppe bedeuten.

Die Gruppen bei R₁ und R₂ können unsubstituiert oder ein- oder mehrfach substituiert sein. Beispiele von Substituenten an den Gruppen bei R₁ und R₂ sind vor allem C₁-C₄-Alkyl, C₁-C₄-Alkoxy, C₁-C₄-Alkylthio, C₁-C₄-Alkoxy-C₁-C₄-Alkyl, wie Aethoxymethyl, C₁-C₄-Alkylthio-C₁-C₄-Alkyl, wie Aethylthiomethyl, 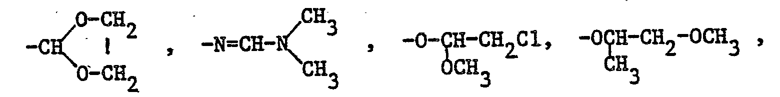 Halogen, Amino, Methylamino, Dimethylamino, Cyano oder Nitro. Die Phenylgruppe kann auch in 2,3- oder 3,4-Stellung, insbesondere in 2,3-Stellung, durch die Gruppen 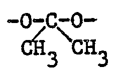 oder -O-CH₂-O-substituiert sein. Unter Halogen ist Fluor, Chlor, Brom oder Jod, insbesondere aber Chlor, zu verstehen.

Die Verbindungen der Formel I können nach an sich bekannten Methoden z.B. wie folgt hergestellt werden: 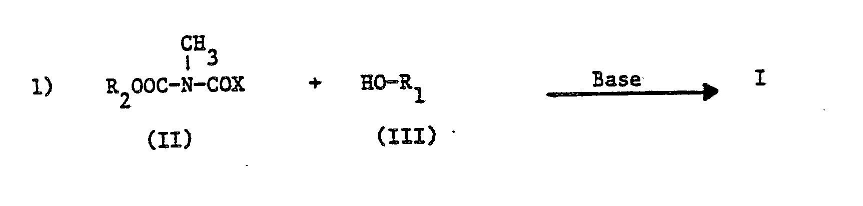 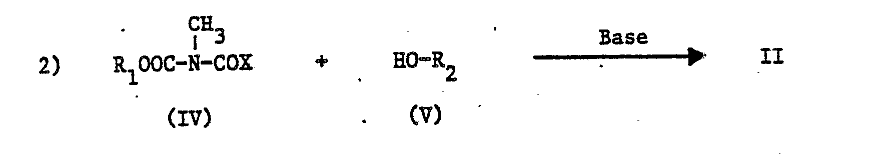 In den Formeln II, III, IV und V haben R₁ und R₂ die für die Formel I angegebene Bedeutung. In den Formeln II und IVsteht X für ein Halogenatom, insbesondere für ein. Chloratom .

Die Verfahren werden bei einer Reaktionstemperatur zwischen -50°C und +130°C, vorzugsweise zwischen -10°C und +100°C, bei normalem oder leicht erhöhtem Druck und in Gegenwart eines gegenüber den Reaktionsteilnehmern inerten Lösungs- oder Verdünnungsmittels vorgenommen.

Als geeignete Basen für die Verfahren kommen insbesondere tertiäre Amine, wie Trialkylamine, Pyridine und Dialkylaniline ferner Hydroxide, Oxide, Carbonate und Bicarbonate von Alkali- und Erdalkalimetallen sowie Alkalimetallalkoholate wie z.B. Kalium-tert.butylat und Natriummethylat in Betracht.

Als Lösungs- oder Verdünnungsmittel eignen sich z.B. Aether und ätherartige Verbindungen wie Diäthyläther, Diisopropyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die Ausgangsstoffe der Formeln II bis V sind bekannt bzw. können analog bekannten Methoden hergestellt werden.

Die Verbindungen der Formel I eignen sich zur Bekämpfung von Schädlingen an Tieren und Pflanzen.

Insbesondere eignen sich die Verbindungen der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina.

Vor allem eignen sich die Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

In diesem Zusammenhang ist hervorzuheben, dass die genannten Verbindungen sich sowohl durch eine stark ausgeprägte systemisch als auch Kontakt-Wirkung gegen saugende Insekten, insbesondere gegen saugende Insekten der Ordnung Homoptera und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z.B. Musca domestica und Mückenlarven. Daneben zeichnen sich die Verbindungen der Formel I durch eine breite ovizide und ovilarvizide Wirkung aus.

Darüberhinaus besitzen die Verbindungen der Formel I fungizide und pflanzenregulatorische Eigenschaften und eine wertvolle Wirkung gegen pflanzenparasitäre Nematoden sowie gegen ektoparasitäre Milben und Zecken, z.B. der Familien Ixodidae, Argasidae und Dermanyssidae.

Die Verbindungen der Formel I werden in unveränderter Form oder vorzugsweise zusammen mit den in der Formulieruagstechnik üblichen Hilfsmitteln eingesetzt und werden daher z.B. zu Emulsionskonzentraten, direkt versprühbaren oder verdünnbaren Lösungen, verdünnten Emulsionen, Spritzpuivern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel 1 und gegebenenfalls einen festen oder flüssigen Zusatzstoff enthaltenden Mittel, Zubereituagen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen-(Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche. Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren (C₁₀-C₂₂), wie z.B.die .Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die-Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyg1ykol- ätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpoly- äthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt..

Ferner kommen auch Fettsäureester von Polyoxyäthylensorbitan wie das Polyoxyäthylensorbitan-trioleat in Betracht.

Bei den kationischen Tensiden handelt es sich vor allem um quartäre.Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Dia Salze liegen vorzugsweise als Balogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:
"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer,Viskositätsregulatoren, Bindemittel, Haftmittel sowie Dünger oder andere Wirkstoffe zur Erzielung spezieller Effekte enthalten.

### Formulierungsbeispiele für flüssige Wirkstoffe der Formel I

### (% = Gewichtsprozent)

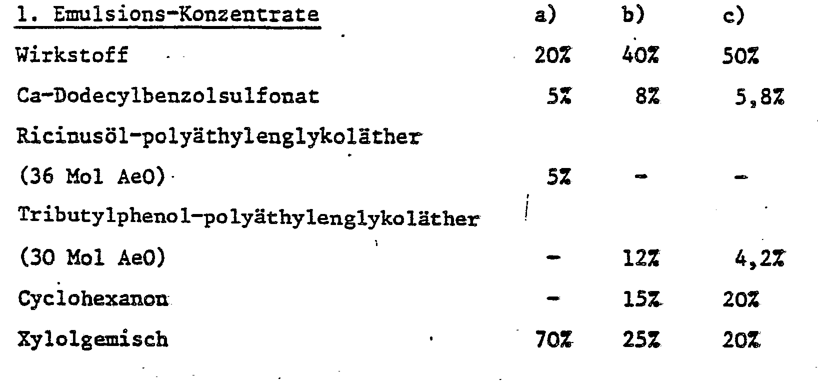

### Aus solchen Konzentraten können durch Verdünnen mit Wasser Emulsionen jeder gewünschten Konzentration hergestellt werden.

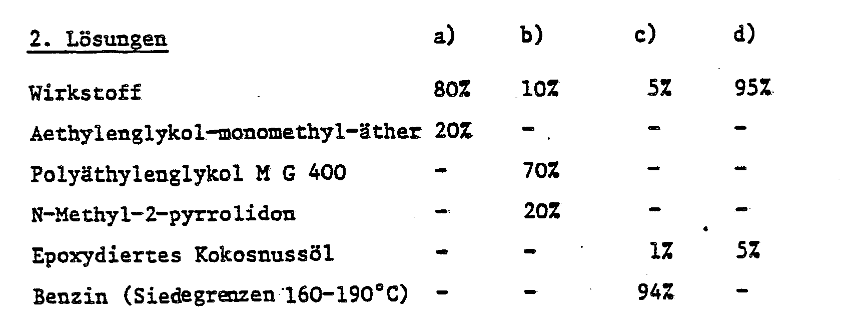

Die Lösungen sind zur Anwendung in Form kleinster Tropfen geeignet.

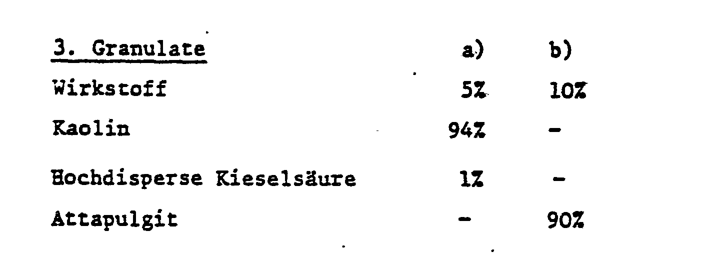

Der Wirkstoff wird in Methylenchlorid gelöst, auf den Träger aufgesprüht und das Lösungsmittel anschliessend im Vakuum abgedampft.

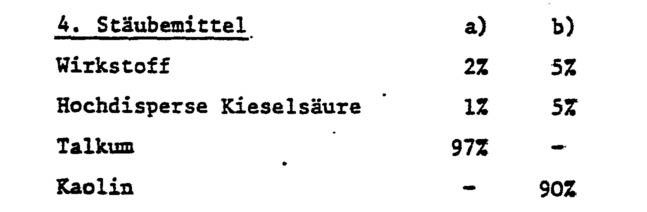

Durch inniges Vermischen der Trägerstoffe mit dem Wirkstoff erhält man gebrauchsfertige Stäubemittel.

### Formulierungsbeispiele für feste Wirkstoffe der Formel I

### (% = Gewichtsprozent)

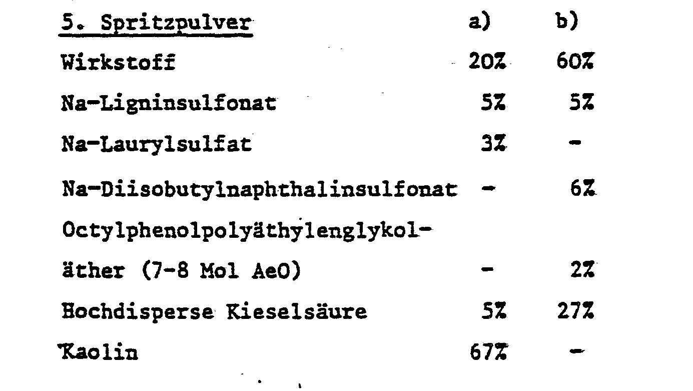

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

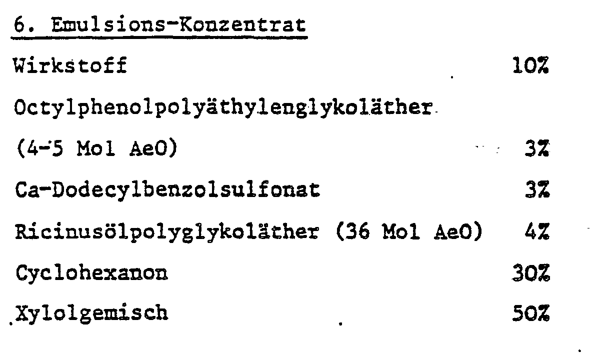

Aus diesem Konzentrat können durch Verdünnen mit Wasser Emulsionen jeder gewünschten Konzentration hergestellt werden.

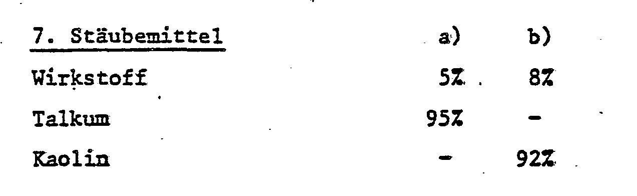

Man erhält anwendungsfertige Stäubemittel, indem der Wirkstoff mit dem Träger vermischt und auf einer geeigneten Mühle vermahlen wird.

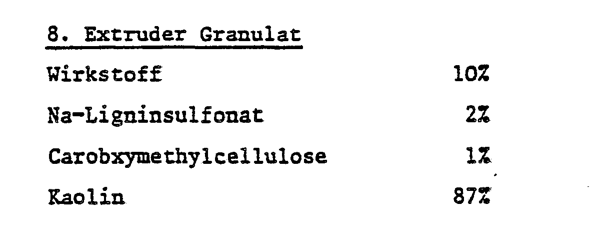

Der Wirkstoff wird mit den Zusatzstoffen vermischt, vermahlen und mit Wasser angefeuchtet. Dieses Gemisch wird extrudiert und anschliessend im Luftstrom getrocknet.

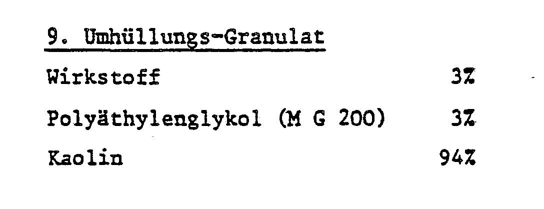

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

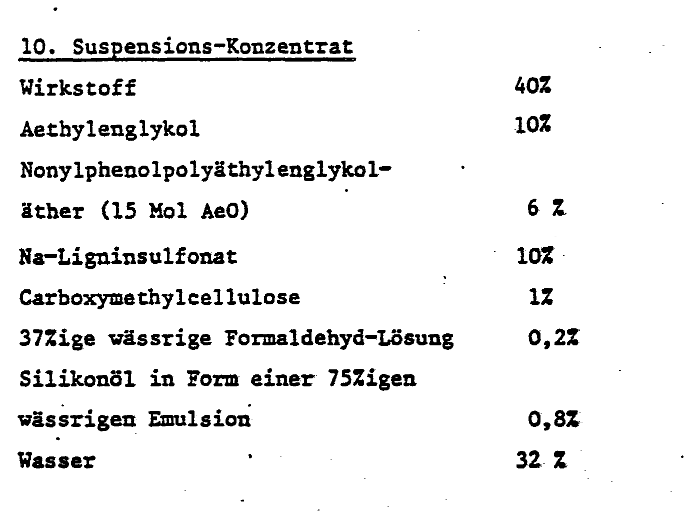

Der fein gemahlene Wirkstoff wird mit den Zusatzstoffen innig vermischt. Man erhält so ein Suspensions Konzentrat, aus welchem durch Verdünnen mit Wasser Suspensionen jeder gewünschten Konzentration hergestellt werden können.

### Beispiel 1: Herstellung von Benzyl-(2,2-dimethyl-2,3-dihydrobenzofuranyl)-N-methyl-bis-carbamat.

Zu einer Lösung von 9,93 g Chlorcarbonyl-M₋methyl-(2,2-dimethyl-2,3-dihydrobenzofuranyl)-carbamat in 100 ml Toluol werden bei 0°C zuerst 3,78 g Benzylalkohol und anschliessend 4,85 ml Triäthylamin zugetropft. Das Reaktionsgemisch wird acht Stunden bei 20°C und zwei Stunden bei 50°C gerührt, dann dreimal mit je 100 ml Wasser gewaschen. Die organische Schicht wird abgetrennt und mit.Natriumsulfat getrocknet. Nach dem Abdestillieren des Toluols erhält man die Verbindung der Formel 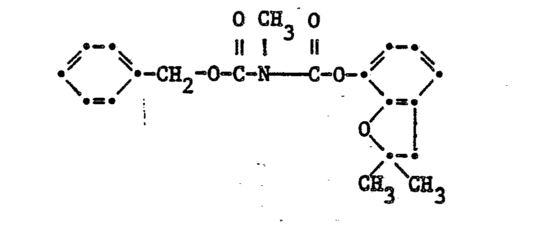 mit einer Refraktion von 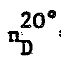 = 1,5497.

Auf dieselbe Art werden auch folgende Substanzen hergestellt:

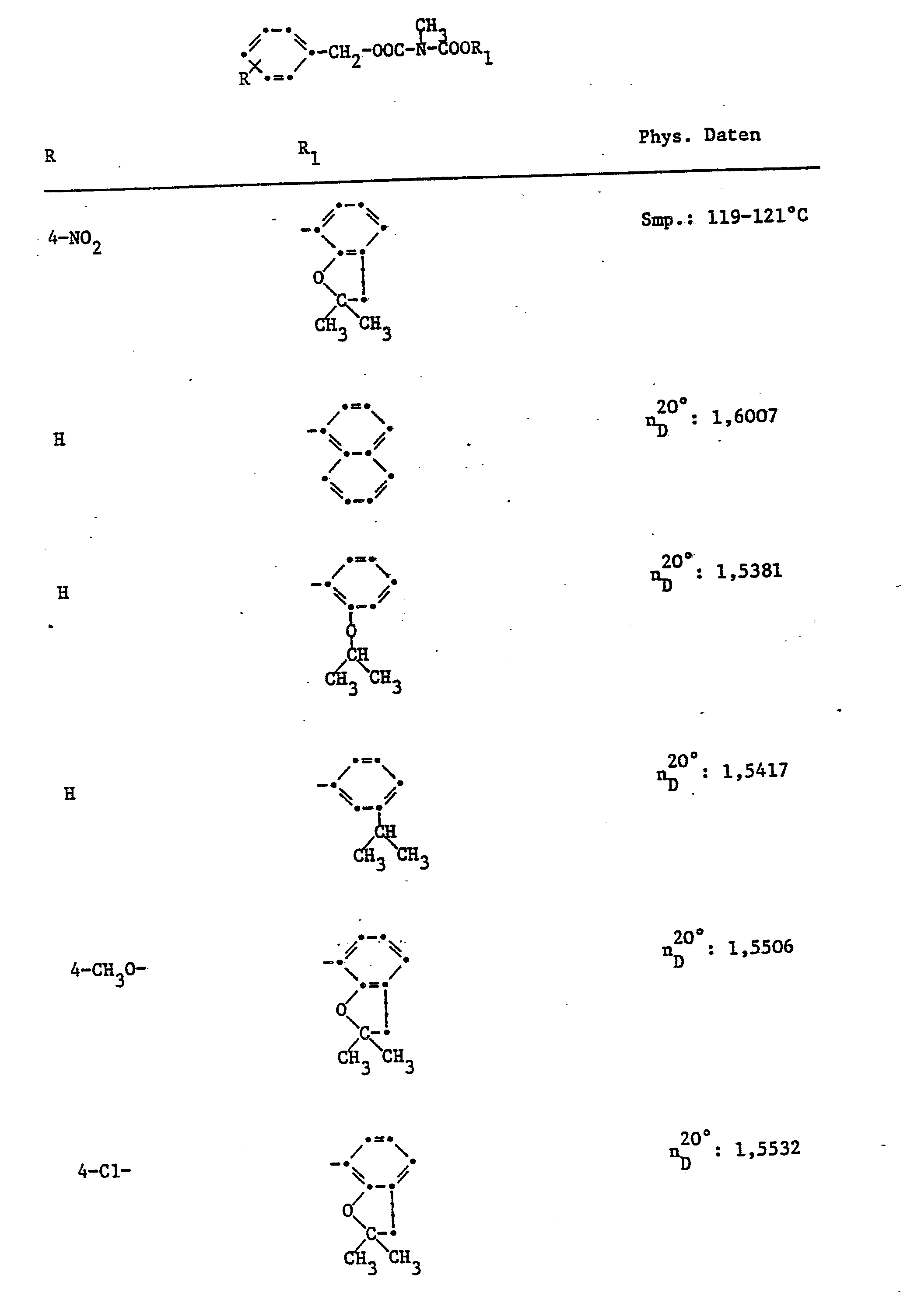

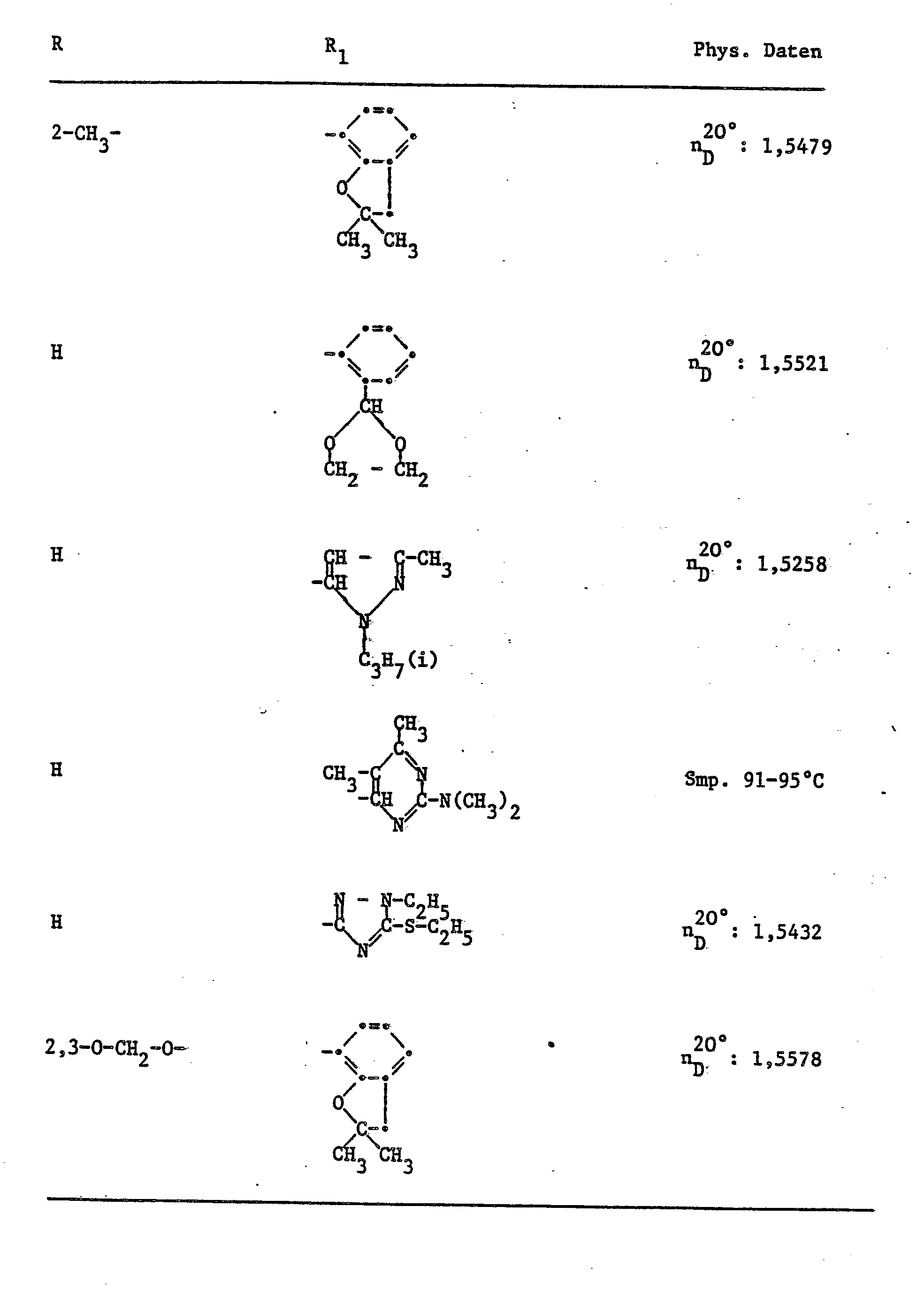

Beispiel 2: Insektizide Frassgift-Wirkung: Laspeyresia pomonella

Diätwürfel werden mit einer Versuchslösung, enthaltend die angegebene Konzentration der zu prüfenden Verbindung, besprüht.

Nach dem Antrocknen des Belages werden die Diätwürfel mit Larven der Spezies Laspeyresia pomonella (L₁-Stadium) besetzt. Man verwendet pro Versuchsverbindung und pro Test-Spezies zwei Pflanzen, und eine Auswertung der erzielten Abtötungsrate erfolgt nach 48 Stunden. Der Versuch wird bei 24°C und 60X relativer Luftfeuchtigkeit durchgeführt.

Die Verbindungen gemäss dem Herstelluagsbeispiel 1 zeigen die in der folgenden Tabelle angegebene Wirkung gegen Larven der Spezies Laspeyresia pomonella.

### Beispiel 3: Insektizide Kontakt-Wirkung: Myzus persicae

In Wasser angezogene Pflanzen (Vicia faba) werden vor dem Versuchsbeginn je mit ca. 200 Individuen der Spezies Myzus persicae besiedelt. Die so behandelten Pflanzen werden 3 Tage später mit einer Lösung enthaltend 10 oder 1 ppm der zu prüfenden Verbindung aus 30 cm Distanz bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

Verbindungen gemäss Beispiel 1 haben die in der folgenden Tabelle angegebene Wirkung gegen Insekten der Spezies Myzus persicae.

Beispiel 4: Insektizide systemische Wirkung: Aphis craccivora

Bewurzelte Bohnenpflanzen werden in Töpfe, welche 600 ccm Erde enthalten, verpflanzt. Anschliessend werden 50 ml einer Versuchslösung enthaltend 25 ppm, 5 ppm bzw. 1 ppm der zu prüfenden Verbindung direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 24 und 48 Stunden nach Versuchsbeginn. Pro Konzentrationsdosis Testsubstanz werden zwei Pflanzen, je eine in einem separaten Topf, verwendet. Der Versuch wurde bei 25°C und 70% relativer Luftfeuchtigkeit durchgeführt.

f Verbindungen gemäss Beispiel 1 haben die in der folgenden Tabelle angegebene Wirkung gegen Insekten der Spezies Aphis craccivora.

### Biologische Versuchsergebnisse

In der folgenden Tabelle sind Versuchsergebnisse auf der Basis der vorstehenden Beispiele aufgeführt, und zwar mit folgendem Bewertungsindex in Bezug auf die prozentuale Abtötung der Schädlinge:

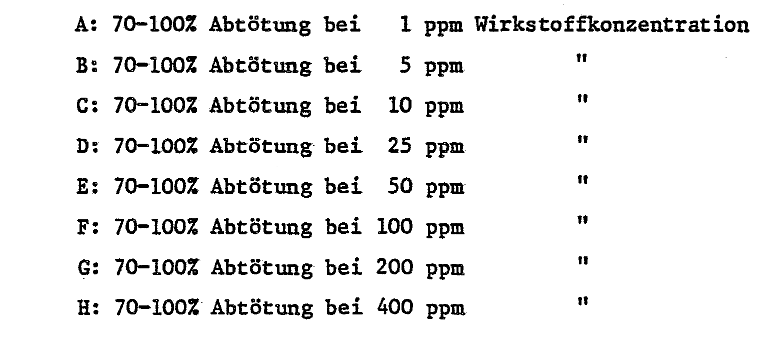

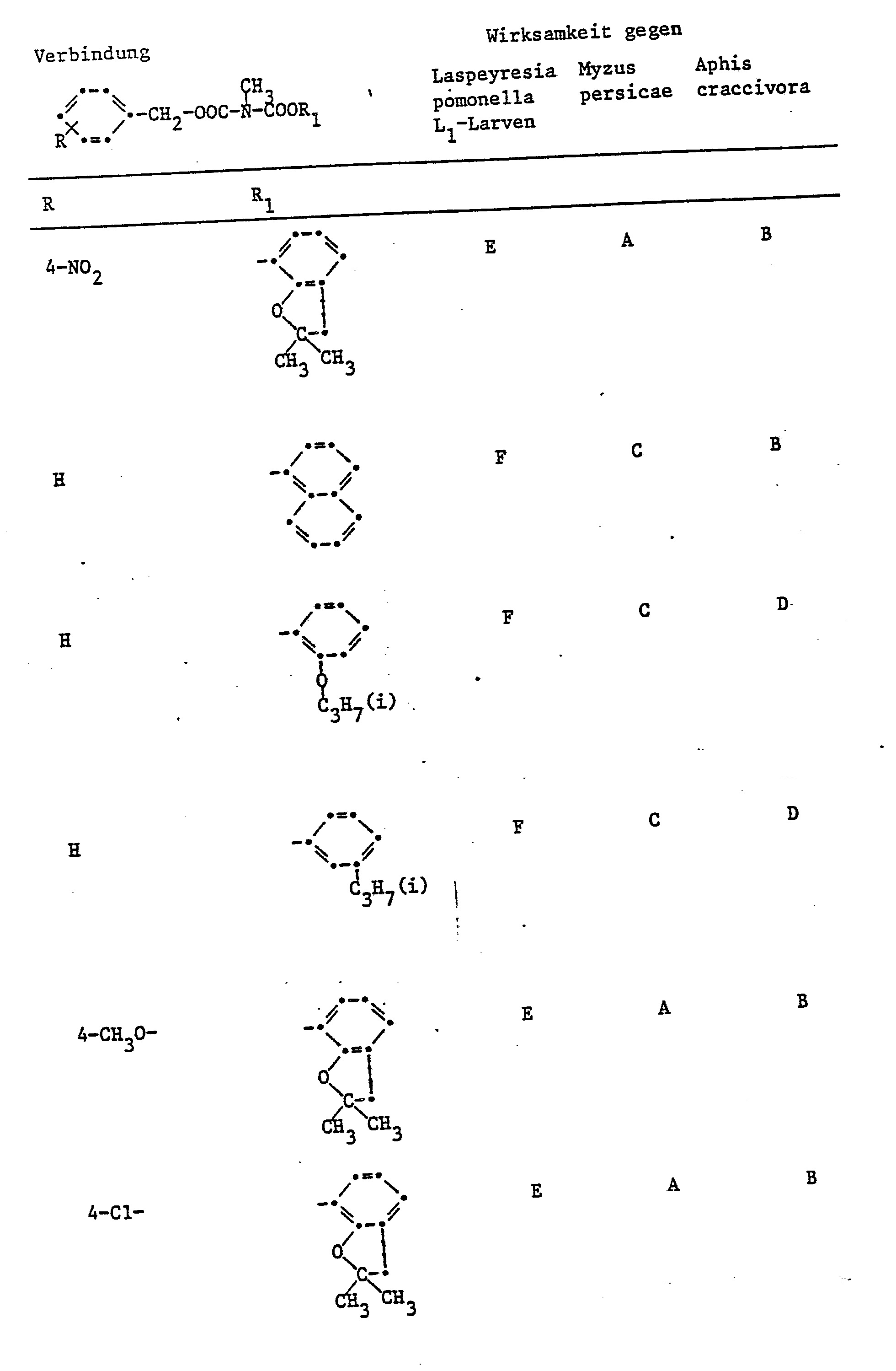

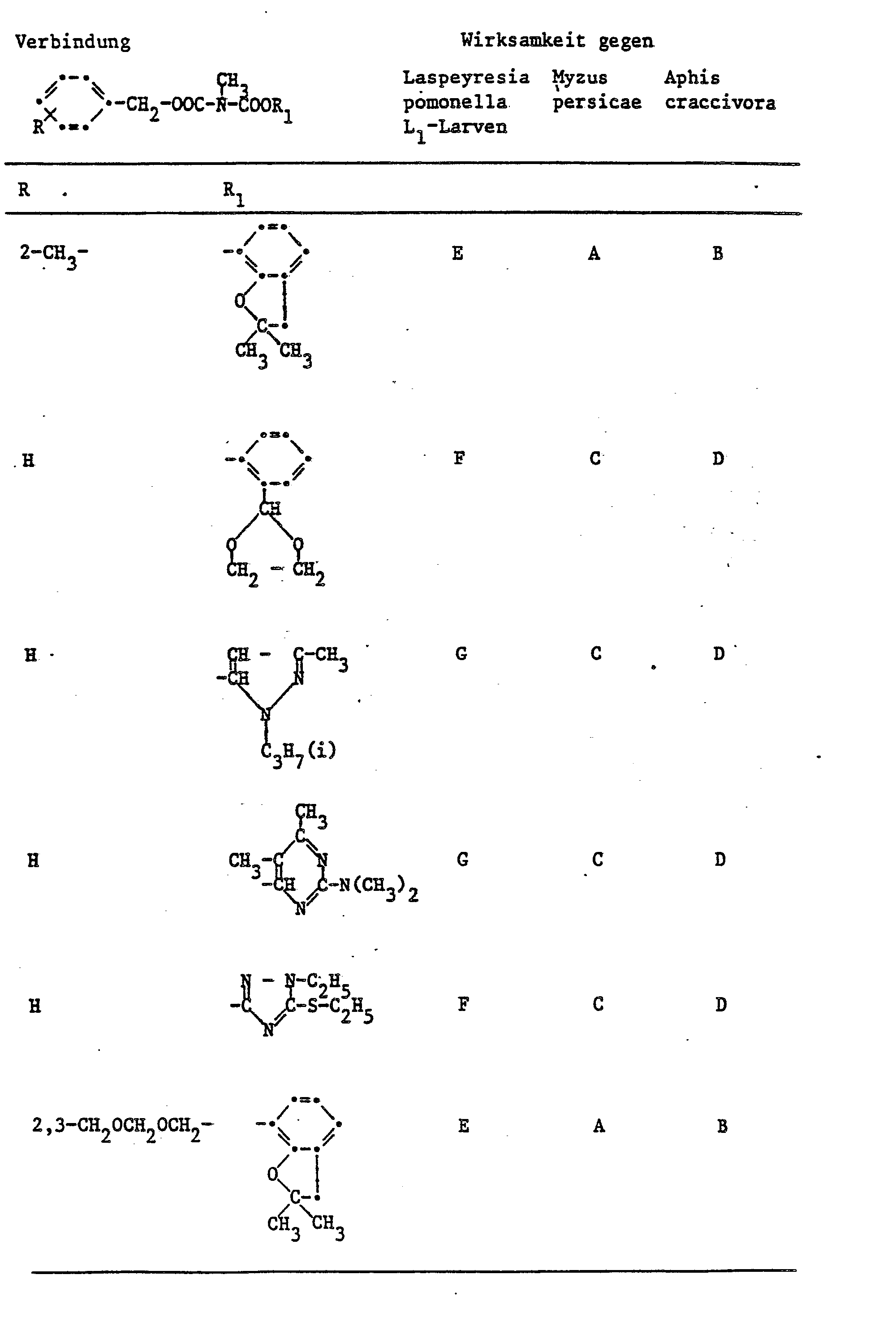

## Patentansprüche

1. Eine Verbindung der Formel 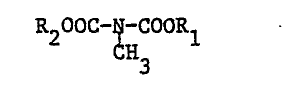 worin R₁ eine Phenyl-, Dihydrobenzofuranyl-, Naphthyl-, Pyrimidyl-, Pyrazolyl-, Triazolyl-, Chinolynyl- oder Tetrahydrochinolinylgruppe und R₂ eine Benzylgruppe bedeuten.

2. Eine Verbindung gemäss Anspruch 1, worin R₁ 2,2-Dimethyl-2,3-dihydrobenzofuranyl, Naphthyl, unsubstituiertes oder ein-oder mehrfach durch C₁-C₄-Alkyl, C_{I}-C₄₋Alkoxy, C₁-C₄-Alkoxy-C₁-C₄-Alkyl, C₁-C₄-Alkyl- thio-C₁-C₄-Alkyl, Dimethylamino, 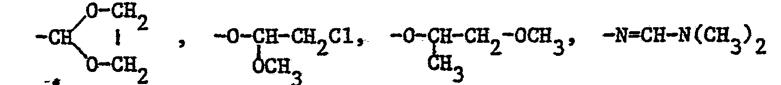 oder in 2,3-Stellung durch 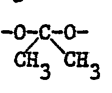 substituiertes Phenyl oder R₂ unsubstituiertes oder ein-oder mehrfach durch Halogen, C₁-C₄-Alkyl, C₁-C₄-Alkoxy, Nitro oder in 2,3-Stellung durch -O-CH₂-O- substituiertes Benzyl bedeuten.

3. Eine Verbindung gemäss Anspruch 1, worin R₁ 2,2-Dimethyl-2,3-dihydrobenzofuranyl, Naphthyl, unsubstituiertes oder ein-, zwei- oder dreifach durch C₁-C₄-Alkyl, C₁-C₄-Alkoxy, Aethylthiomethyl, Aethoxymethyl, Dimethylamino, 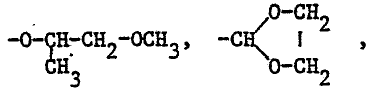 -N=CH-N(CH₃)₂-oder in 2,3-Stellung durch 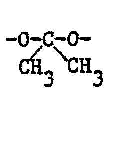 substituiertes Phenyl und R₂ unsubstituiertes oder ein-oder mehrfach durch Halogen, Nitro, Methyl, Methoxy oder in 2,3-Stellung durch -O-CH₂-O- substituiertes Benzyl bedeuten.

4. Eine Verbindung gemäss Anspruch 3, worin R₁ 2,2-Dimethyl-2,3-dihydrobenzofuranyl und R₂ unsubstituiertes oder einfach durch Chlor, Methyl, Methoxy, Nitro oder in 2,3-Stellung durch -O-CH₂-O- substituiertes Benzyl bedeuten.

5. Die Verbindung gemäss Anspruch 4 der Formel

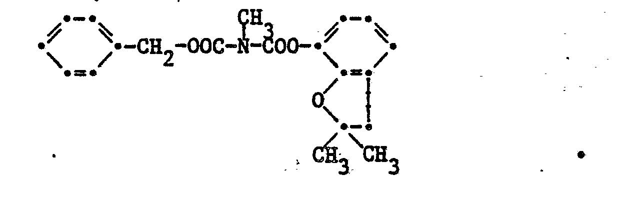

6. Die Verbindung gemäss Anspruch 4 der Formel

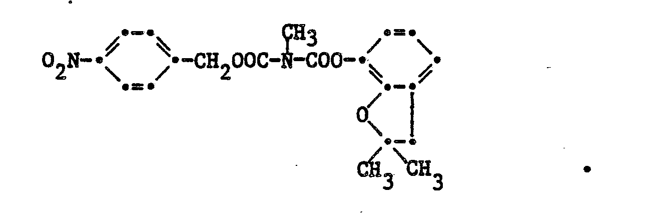

7. Die Verbindung gemäss Anspruch 4 der Formel

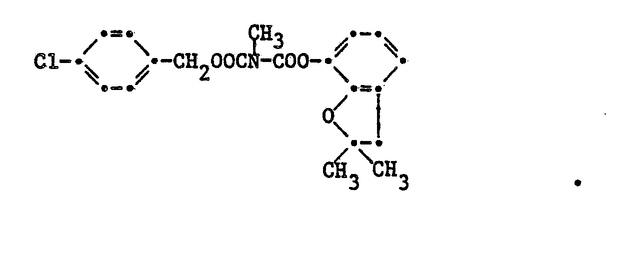

8. Die Verbindung gemäss Anspruch 4 der Formel

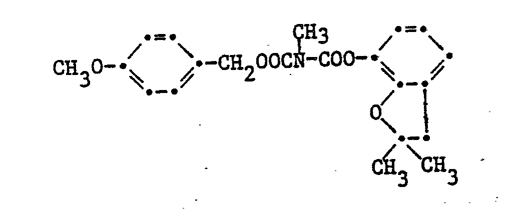

9. Die Verbindung gemäss Anspruch 4 der Formel

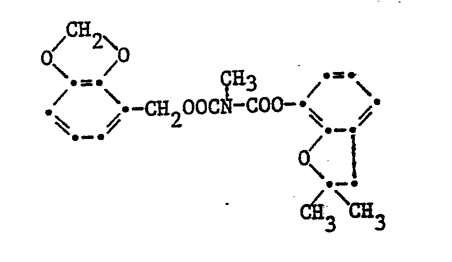

10. Die Verbindung gemäss Anspruch 4 der Formel

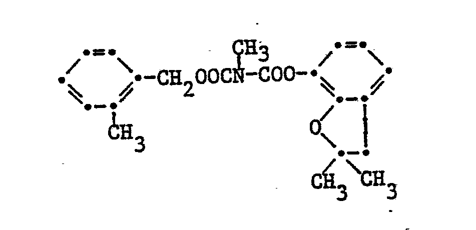

11. Die Verbindung gemäss Anspruch 3 der Formel

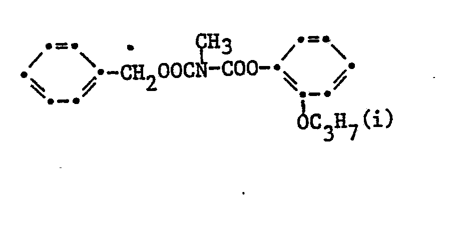

12. Die Verbindung gemäss Anspruch 3 der Formel

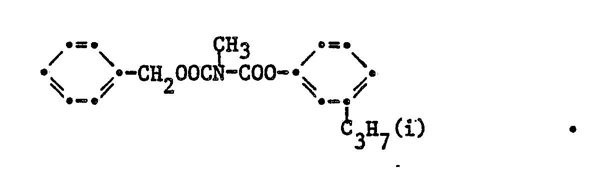

13. Die Verbindung gemäss Anspruch 3 der Formel

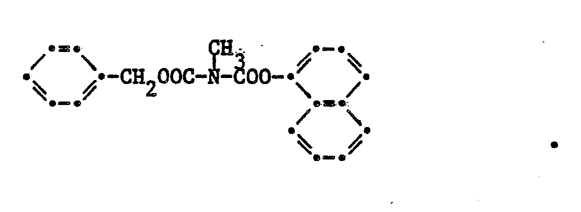

14. Verfahren zur Herstellung von Verbindungen gemäss Anspruch 1, dadurch gekennzeichnet, .dass man in Gegenwart einer Base eine Verbindung 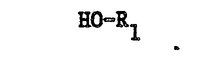 mit einer Verbindung der Formel 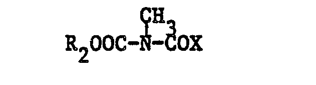 umsetzt, worin R₁ und R₂ die in Anspruch 1 angegebene Bedeutung haben und X für ein Halogenatom steht.

15. Ein Schädlingsbekämpfungsmittel, welches als aktive Komponente eine Verbindung gemäss Anspruch 1 enthält.

16. Die Verwendung einer Verbindung gemäss Anspruch 1 zur Bekämpfung von Schädlingen an Tieren und Pflanzen.

17. Die Verwendung gemäss Anspruch 16 zur Bekämpfung von Insekten und Vertretern der Ordnung Akarina.
